# EUROPÄISCHE PATENTANMELDUNG

(11) **EP 2 322 138 A2**
(43) Veröffentlichungstag der Anmeldung: **18.05.2011**
(21) Anmeldenummer: 10173346.7
(22) Anmeldetag: 19.08.2010
(51) Int. Cl.: A61K 8/64, A61K 8/97, A61Q 15/00, A61Q 19/00, A61Q 5/00

(54) **Antimikrobielle Formulierung**

(30) Priorität: 02.09.2009 DE 102009029110
(71) Anmelder: Henkel AG & Co. KGaA, 40589 Düsseldorf (DE)
(72) Erfinder: Janßen, Frank, 41470, Neuss (DE); Banowski, Bernhard, 40597, Düsseldorf (DE); Holtkötter, Olaf, 50354, Hürth (DE)

(57) **Zusammenfassung**

Die Erfindung betrifft eine Zusammensetzung zur Behandlung der Haut und/oder der Haare, enthaltend in einem kosmetisch akzeptablen Medium - bezogen auf ihr Gesamtgewicht -
a) 0,000005 bis 0,005 Gew.-% mindestens einer funktionalisierten Verbindung aus zwei bis sechs über Amidbindungen miteinander verknüpften alpha-Aminosäuren und
b) 0,0001 bis 10 Gew.-% mindestens eines Extrakts aus Pflanzen der Familie der Monimiaceae,

Die Zusammensetzung ist geeignet für die Bekämpfung von Mikroorganismen auf der Haut und/oder den Haaren.

## Beschreibung

Die Erfindung liegt auf dem Gebiet der kosmetischen Mittel und betrifft eine Zusammensetzung zur Behandlung der Haut und/oder der Haare auf der Basis einer bestimmten Wirkstoffkombination. Die Erfindung betrifft weiterhin die Verwendung dieser Wirkstoffkombination in kosmetischen Haut- und/oder Haarbehandlungsmitteln zur Bekämpfung von Mikroorganismen und zur Stimulierung der humanen Hautpeptide β-Defensine-2 und β-Defensine-3 sowie Verfahren zur Haut- und/oder Haarbehandlung unter Verwendung des Mittels.

Die Verwendung antimikrobieller Substanzen in kosmetischen Zusammensetzungen zur Bekämpfung von Keimen, die die Hautflora negativ beeinträchtigen und ursächlich sind für eine Reihe unerwünschter Phänomene wie Pickel, Schuppen, Plaque und/oder Hautgeruch, ist aus dem Stand der Technik bekannt.

Körpergeruch entsteht durch den Abbau von Schweißbestandteilen durch Bakterien der Hautflora. Es ist bekannt, dass viele antibakterielle Wirkstoffe schlecht gegen Körpergeruch wirken. Mikrobielle Aktivität ist auch die Ursache oder ein bedeutender Faktor für die Entwicklung von unreiner Haut bzw. Pickeln sowie Kopfschuppen oder Plaque.

Herkömmliche antimikrobielle Substanzen weisen oftmals den Nachteil auf, dass sie eine schlechte Hautverträglichkeit aufweisen, und neben der erwünschten Beseitigung/Verminderung/Vorbeugung von Keimen häufig auch unerwünschte Hautreaktionen wie beispielsweise Reizungen, Rötungen oder Sensibilisierungsreaktionen zeigen.

Ein weiterer Nachteil einiger herkömmlicher antimikrobieller Substanzen besteht darin, dass sie oft unselektiv auf die Mikroorganismen der Hautflora wirken und nicht nur die schädlichen, sondern auch die nützlichen Keime auf der Haut (insbesondere im Gesicht, auf der Kopfhaut sowie unter den Achseln) hemmen. Die Folge davon ist, dass die Störung der Hautmikroflora nach ggf. kurzfristiger Verbesserung des entsprechenden Hautzustandes häufig nach Absetzen der Anwendung zu einer Neueinstellung des Gleichgewichts der verschiedenen Hautkeime führt, das ggfs. auch hin zu einem verstärkten Auftreten von pathogenen/schädlichen Mikroorganismen verschoben sein kann.

Schließlich kann durch die Behandlung der Haut und/oder der Haare mit antimikrobiellen Mitteln auch die Hautbeschaffenheit negativ beeinflusst werden, insbesondere dann, wenn die Mittel aggressiv sind und die Haut austrocknen.

Es besteht daher der Bedarf nach antimikrobiellen Wirkstoffen bzw. nach kosmetischen Zusammensetzungen auf der Basis von antimikrobiellen Wirkstoffen, die besser und selektiver gegenüber Körpergeruch, Pickel, Plaque und Schuppen wirken als die bislang bekannten Mittel. Es besteht weiterhin der Bedarf nach kosmetischen Zusammensetzungen mit antibakterieller Wirkung, die schädliche Keime auf der Haut bekämpfen, die nützlichen Keime aber weniger stark schädigen bzw. nicht hemmen.

Die kosmetischen Zusammensetzungen mit antimikrobieller Wirkung sollen weiterhin die Haut oder Kopfhaut pflegen und und insbesondere trockener, rauer und rissiger Haut entgegenwirken.

Es wurde gefunden, dass die Kombination eines funktionalisierten Oligopeptids mit einem bestimmten Pflanzenextrakt in einer kosmetischen Zusammensetzung diese Anforderungen erfüllt. Kosmetische Zusammensetzungen auf der Basis dieser Wirkstoffkombination sind selektiv wirksam gegen Bakterien und Keime, die ursächlich sind für Körpergeruch, Pickel, Plaque und/oder Schuppen und die mit den kosmetischen Zusammensetzungen behandelte Haut/Kopfhaut kann wieder in ihr natürliches Gleichgewicht zurückgebracht werden.

Gegenstand der vorliegenden Erfindung sind daher Zusammensetzungen zur Behandlung der Haut und/oder der Haare, die in einem kosmetisch akzeptablen Träger - bezogen auf ihr Gesamtgewicht -
a) 0,000005 bis 0,005 Gew.-% mindestens einer funktionalisierten Verbindung aus zwei bis sechs über Amidbindungen miteinander verknüpften alpha-Aminosäuren und
b) 0,001 bis 10 Gew.-% mindestens eines Extrakts aus Pflanzen der Familie der Monimiaceae.
enthalten.

Geeignete Träger für die erfindungsgemäßen Zusammensetzungen werden an späterer Stelle (unter den jeweils bevorzugten Ausführungsformen) beschrieben.

Die erfindungsgemäßen Zusammensetzungen enthalten mindestens eine funktionalisierte Verbindung a) aus zwei bis sechs über Amidbindungen miteinander verknüpften alpha-Aminosäuren (im Prioritätsdokument als "funktionalisiertes Dipeptid" bezeichnet).

In einer bevorzugten Ausführungsform enthält die funktionalisierte Verbindung a) zwei bis vier und insbesondere zwei alpha-Aminosäuren, die über Amidbindungen miteinander verknüpft sind. Durch die Verknüpfung verbleiben in der Peptid-Hauptkette eine freie Aminogruppe, der sogenannte N-Terminus, sowie eine freie Carboxylgruppe, der sogenannte C-Terminus. An dem N- und/oder dem C-Terminus können Verbindungen a) funktionalisiert werden.

Besonders bevorzugte Verbindungen a) sind solche, die am N-Terminus mit geeigneten Carbon- oder Sulfonsäurederivaten amidiert oder mit Alkylierungsmitteln alkyliert wurden, und am C-Terminus mit Alkoholen verestert oder mit Ammoniak, primären oder sekundären Aminen amidiert wurden.

Ganz besonders bevorzugt sind Verbindungen a), die am N-Terminus mit Carbonsäurederivaten wie beispielsweise Acetylchlorid oder Acetylanhydrid aacyliert, insbesondere acetyliert, und am C-Terminus mit primären Aminen amidiert wurden.

Insbesondere bevorzugt sind Verbindungen a), die am N-Terminus eine Acetylgruppe und/oder am C-Terminus eine Aminohexanoatgruppe tragen.

Beispiele für geeignete Verbindungen a) sind unter der INCI-Bezeichnung "Acetyl Dipeptide-3 Aminohexanoate" bekannt.

Die Verbindungen a) werden in den erfindungsgemäßen Zusammensetzungen - bezogen auf ihr Gesamtgewicht - in Mengen von 0,000005 bis 0,005 Gew.-%, bevorzugt in Mengen von 0,00001 bis 0,004 Gew.-% und insbesondere in Mengen von 0,00005 bis 0,0025 Gew.-% eingesetzt. Alternativ können die erfindungemäßen Zusammensetzungen auch das Handelsprodukt "Bodyfensine^{®}" enthalten. Dieses von der Firma Lipotec im Handel erhältliche Produkt liegt in der Form eines opaken Gels vor und enthält neben Butylene Glycol und Wasser 0,05 Gew.-% Acetyl Dipeptide-3 Aminohexanoate. Das Gel kann den erfindungsgemäßen Zusammensetzungen - bezogen auf deren Gewicht - bevorzugt in Mengen von 0,01 bis 10 Gew.-% und insbesondere in Mengen von 0,1 bis 5 Gew.-% zugegeben werden.

Die erfindungsgemäßen Zusammensetzungen enthalten weiterhin mindestens einen Extrakt aus Pflanzen der Familie der Monimiaceae b).

Diese Pflanzenextrakte können in der Kombination mit der Verbindung a) in der erfindungsgemäßen Zusammensetzung zur Stimulierung der hauteigenen β-Defensine beitragen sowie trockener, rissiger und rauer Haut entgegenwirken und sie geschmeidiger machen. Dadurch wird die Haut weniger sensibel für schädliche Keime.

Monimiengewächse (Monimiaceae) sind eine Familie bedecktsamiger Pflanzen, die in Australien, Südamerika und Südostasien beheimatet sind.

Zur Familie der Monimiengewächse gehören 22 Gattungen mit etwa 200 Arten, aus denen geeignete Pflanzenextrakte erhalten werden können.

Bevorzugt für die Verwendung in den erfindungsgemäßen Zusammensetzungen ist mindestens ein Extrakt aus Pflanzen der Gattung Peumus und der Art Boldo.

Besonders geeignet ist der Extrakt des Peumus Boldus, wobei der Extrakt prinzipiell aus allen Teilen der Pflanze hergestellt werden kann. Bevorzugt sind jedoch Extrakte, die ausschließlich aus den Blättern der Pflanze hergestellt wurden.

Als Extraktionsmittel zur Herstellung der genannten Pflanzenextrakte können Wasser, Alkohole sowie deren Mischungen verwendet werden. Unter Alkoholen sind dabei niedere Alkohole wie Ethanol und Isopropanol, insbesondere aber mehrwertige Alkohole wie Ethylenglykol, Propylenglykol, Butylenglycol und/oder Glycerin, sowohl als alleiniges Extraktionsmittel als auch in Mischung mit Wasser, bevorzugt. Pflanzenextrakte aus den Blättern des Peumus Boldus (Boldobaums) auf Basis von Wasser/Butylenglycol im Verhältnis 1:10 bis 10:1 haben sich als besonders geeignet erwiesen.

Der Pflanzenextrakt kann sowohl in reiner als auch in verdünnter Form eingesetzt werden. Sofern er in verdünnter Form eingesetzt wird, enthält er üblicherweise ca. 2 - 80 Gew.-% Aktivsubstanz und als Lösungsmittel das bei seiner Gewinnung eingesetzte Extraktionsmittel oder Extraktionsmittelgemisch.

Der Pflanzenextrakt b) wird in den erfindungsgemäßen Zusammensetzungen - bezogen auf deren Gesamtgewicht - in einer Menge von 0,0001 bis 10 Gew.-%, bevorzugt von 0,0005 bis 8 Gew.-%, mehr bevorzugt von 0,001 bis 5 Gew.-% und insbesondere von 0,005 bis 2 Gew.-% eingesetzt. Alternativ können die erfindungemäßen Zusammensetzungen anstatt des reinen Boldobaum-Blätterextrakts auch das Handelsprodukt "Betapur^{®}" enthalten. Dieses von der Firma BASF im Handel erhältliche Produkt kann den erfindungsgemäßen Zusammensetzungen - bezogen auf deren Gewicht - bevorzugt in Mengen von 0,01 bis 10 Gew.-% und insbesondere in Mengen von 0,1 bis 5 Gew.-% zugegeben werden.

Gemäss einer ersten bevorzugten Ausführungsform können die erfindungsgemäßen Zusammensetzungen neben den Wirkstoffen a) und b) weiterhin mindestens einen Sebumregulator enthalten.

Insbesondere in kosmetischen Zusammensetzungen für die unreine Haut bzw. für die Behandlung von Akne ist der Zusatz von Sebumregulatoren bevorzugt.

Aber auch in komsetischen Zusammensetzungen für die Behandlung der Haare wie Haarpflegemitteln, Haarshampoos und/oder Haarstylingmitteln kann ein Zusatz mindestens eines Sebumregulators vorteilhaft sein, denn dieser kann in Kombination mit den Wirkstoffen a) und b) der Entstehung fettiger Haare und/oder Schuppenbildung auf der Kopfhaut entgegenwirken. Besonders geeignete sebumregulatorische Wirkstoffe sind:
Azelainsäure, Sebacinsäure, 10-Hydroxydecansäure, 1,10-Decandiol (insbesondere bevorzugt ist die Dreierkombination Sebacinsäure, 10-Hydroxydecansäure, 1,10-Decandiol, wie beispielsweise Acnacidol PG (Vincience), Azeloglicina (Potassium Azeloyl Diglycinate, Sinerga), Extrakten aus Spiraea Ulmaria (wie z. B. im Produkt Seboregul der Firma Silab enthalten).

Weiterhin bevorzugt sind wasser- und öllösliche Extrakte aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques), Asebiol^{®} BT 2 (Laboratoires Sérobiologiques, INCI: Aqua, Hydrolyzed Yeast Protein, Pyridoxine, Niacinamide, Glycerin, Panthenol, Biotin), Antifettfaktor^{®} COS-218/2-A (Cosmetochem, INCI: Aqua, Cetyl-PCA, PEG-8 Isolauryl Thioether, PCA, Cetyl Alcohol).

Gemäss einer zweiten bevorzugten Ausführungsform können die erfindungsgemäßen Zusammensetzungen neben den Wirkstoffen a) und b) weiterhin mindestens einen antiinflammatorischen und/oder hautberuhigenden Wirkstoff enthalten.

Besonders geeignete antiinflammatorische Wirkstoffe sind ausgewählt aus:
- Silymarin Phytosome (INCI: Silybum Marianum Extract and Phospholipids) von der Firma Indena SpA, Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter der Handelsbezeichnung Calmsphere von Soliance erhältlich ist,
- Mischungen aus Getreidewachsen, Extrakten aus Schibutter und Argania Spinosa-Öl mit der INCI-Bezeichnung "Spent grain wax and butyrospermum parkii (shea butter) extract and Argania Spinosa Kernel Oil", wie sie z. B. unter der Handelsbezeichnung Stimu-Tex AS von der Firma Pentapharm erhältlich ist,
- Extrakten aus Vanilla Tahitensis, wie sie z. B. unter der Handelsbezeichnung Vanirea (INCI : Vanilla Tahitensis Fruit Extract) von der Firma Solabia erhältlich ist,
- Extrakten aus Olivenblättern (INCI: Olea Europaea (Olive) Leaf Extract), wie sie insbesondere unter der Handelsbezeichnung Oleanoline DPG von der Firma Vincience erhältlich sind,
- weiterhin Alginhydrolysaten, wie sie z. B. unter der Handelsbezeichnung Phycosaccharide, insbesondere Phycosaccharide AI, von der Firma Codif erhältlich sind,
- Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter der Handelsbezeichnung Rooibos Herbasec MPE von der Firma Cosmetochem erhältlich sind,
- den physiologisch verträglichen Salzen von Sterolsulfaten, wie sie z. B. unter der Handelsbezeichnung Phytocohesine (INCI: Sodium Beta-Sitosterylsulfate) von der Firma Vincience erhältlich sind, sowie
- α-Bisabolol,
- α-Liponsäure,
- Panthenol oder
- Fucogel 100 (Solabia).
Vorteilhafterweise ermöglicht die Kombination der Wirkstoffe a) und b) mit mindestens einem der zuvor genannten antiinflammatorisch wirksamen Stoffe in den erfindungsgemäßen Zusammensetzungen eine besonders schonende, nicht- oder wenig reizende Behandlung empfindlicher Haut, trockener Haut, atopischer Dermatitis, Altershaut, UV-geschädigter Haut und/oder gereizter Haut.

Bei der erfindungsgemäßen Zusammensetzung kann es sich um jede beliebige Darreichungsform handeln, beispielsweise um eine feste oder flüssige Seife, eine Lotion, ein Spray, eine Creme, ein Gel, eine Emulsion, eine Reinigungsflüssigkeit oder Reinigungsmilch, ein Deodorant, ein Antitranspirant, eine Salbe, eine Paste, eine Haarkur oder ein Shampoo und sie kann auch in jeder der beschriebenen oder sonstigen Darreichungsformen enthalten sein, beispielsweise auch in einem Pflaster, insbesondere in einem Gel-Reservoir- oder Matrixpflaster.

In einer bevorzugten Ausführungsform handelt es sich bei der erfindungsgemäßen Zusammensetzung um ein Wasch- und/oder Reinigungsmittel für die Haut und/oder die Haare, ein Deodorant, ein Mund- und/oder Zahnpflegemittel, ein Hautpflegemittel und/oder ein Haarstylingmittel.

In einer bevorzugten Ausführungsform der Erfindung liegen die Zusammensetzungen als Emulsion vor und können für die Pflege der Haut verwendet werden. Innerhalb dieser Ausführungsform enthalten die erfindungsgemäßen Zusammensetzungen bevorzugt mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und - oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.

Die erfindungsgemäßen Mittel enthalten die Emulgatoren bevorzugt in Mengen von 0,1 bis 25 Gew.-%, insbesondere 0,5 - 15 Gew.-%, bezogen auf das gesamte Mittel.

In einer besonders bevorzugten Ausführungsform ist mindestens ein nichtionischer Emulgator mit einem HLB-Wert von 8 und darunter enthalten. Derart geeignete Emulgatoren sind beispielsweise Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.

Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden. Weitere geeignete Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

In einer weiteren bevorzugten Ausführungsform handelt es sich bei der erfindungsgemäßen Zusammensetzung um ein Deodorant und/oder Antitranspirant. Das Deodorant und/oder Antitranspirant liegt hierbei vorzugsweise als Puder, in Stiftform, als Syndet, Waschlotion, Aerosolspray, Pumpspray, flüssige oder gelförmige Roll-on-Applikation, Creme, Schaum, flüssige oder feste Seife, Gel oder als getränktes flexibles Substrat vor.

Als Applikatoren können entsprechend je nach Anwendungsform beispielsweise Stifthülse, Roll-on, Pumpe, Tube, Tiegel, Spender, Tuch, Aerosoldose oder Flasche verwendet werden. Als Applikationsort kommt die Haut jedes Körperbereichs in Frage, insbesondere die Gesichtshaut, die Kopfhaut, die Haut auf den Füßen und den Händen sowie der Mundschleimhaut und der vaginalen Mucosa. In einer weiteren besonders bevorzugten Ausführungsform handelt es sich bei dem Applikationsort um die Haut im Axillarbereich.

Deodorant- oder Antitranspirant-Zusammensetzungen können weiterhin Fettstoffe enthalten. Unter Fettstoffen sind Fettsäuren, Fettalkohole, natürliche und synthetische kosmetische Ölkomponenten sowie natürliche und synthetische Wachse zu verstehen, die sowohl in fester Form als auch flüssig in wässriger oder öliger Dispersion vorliegen können.

Als Fettsäuren können eingesetzt werden lineare und/oder verzweigte, gesättigte und/ oder ungesättigte C₈₋₃₀-Fettsäuren. Bevorzugt sind C₁₀₋₂₂-Fettsäuren. Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt ist der Einsatz von Stearinsäure. Die eingesetzten Fettsäuren können eine oder mehrere Hydroxygruppen tragen. Bevorzugte Beispiele hierfür sind die α-Hydroxy-C₈-C₁₈-Carbonsäuren sowie 12-Hydroxystearinsäure. Die Einsatzmenge beträgt dabei bevorzugt 0,5 - 10 Gew.-%, besonders bevorzugt 1 - 5 Gew.-%, jeweils bezogen auf die gesamte Zusammensetzung.

Als Fettalkohole können eingesetzt werden gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar sind z.B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole. Für Stiftformulierungen werden häufig Wachse verwendet. Als natürliche oder synthetische Wachse können eingesetzt werden feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.

Weiterhin geeignet sind die Mono-, Di- und Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glycerylmonostearat (Cutina^{®} MD), Glyceryltribehenat oder Glyceryltri-12-hydroxystearat, weiterhin synthetische Vollester aus Fettsäuren und Glykolen (z. B. Syncrowachs^{®}) oder Polyolen mit 2 - 6 C-Atomen, Fettsäuremonoalkanolamide mit einem C₁₂₋₂₂-Acylrest und einem C₂₋₄-Alkanolrest, Estern aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/ oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen, sofern die einzelnen Wachskomponenten oder ihre Mischung bei Raumtemperatur fest sind.

Besonders bevorzugt ist, die Wachskomponenten zu wählen aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen, sofern die Wachskomponente oder die Gesamtheit der Wachskomponenten bei Raumtemperatur fest sind. Insbesondere vorteilhaft können die Wachskomponenten aus der Gruppe der C₁₆₋₃₆-Alkylstearate, der C₂₋₄₀-Alkylisostearate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse, zum Beispiel Stearyltrimethylsilan/Stearylalkohol sind gegebenenfalls vorteilhaft. Besonders bevorzugte Wachskomponenten sind die Ester aus gesättigten, einwertigen C₂₀-C₆₀-Alkoholen und gesättigten C₈-C₃₀-Monocarbonsäuren, insbesondere ist ein C₂₀-C₄₀-Alkylstearat bevorzugt, das unter dem Namen Kesterwachs^{®} K82H von der Firma Koster Keunen Inc. erhältlich ist. Das Wachs oder die Wachskomponenten sollten bei 25° C fest sein, jedoch im Bereich von 35 - 95°C schmelzen, wobei ein Bereich von 45 - 85 °C bevorzugt ist.

Natürliche, chemisch modifizierte und synthetische Wachse können alleine oder in Kombination eingesetzt werden.

Die Wachskomponenten sind vorzugsweise in einer Menge von 0,1 bis 40 Gew.-%, bezogen auf die Gesamtzusammensetzung, und insbesondere in einer Menge von 5 - 15 Gew.-% enthalten.

Die erfindungsgemäßen Zusammensetzungen können weiterhin wenigstens ein unpolares oder polares flüssiges Öl, das natürlich oder synthetisch sein kann, enthalten. Die polare Ölkomponente kann ausgewählt sein aus pflanzlichen Ölen, z. B. Sonnenblumenöl, Olivenöl, Sojaöl, Rapsöl, Mandelöl, Jojobaöl und den flüssigen Anteilen des Kokosöls sowie synthetischen Triglyceridölen, aus Esterölen, das heißt den Estern von C₆-₃₀-Fettsäuren mit C₂₋₃₀-Fettalkoholen, aus Dicarbonsäureestern wie Di-n-butyladipat, Di-(2-ethylhexyl)-adipat und Di-(2-ethylhexyl)-succinat sowie Diolestern wie Ethylenglykoldioleat und Propylenglykoldi(2-ethylhexanoat), aus symmetrischen, unsymmetrischen oder cyclischen Estern der Kohlensäure mit Fettalkoholen, Glycerincarbonat oder Dicaprylylcarbonat (Cetiol^{®} CC), aus Mono,- Di- und Trifettsäureestern von gesättigten und/oder ungesättigten linearen und/oder verzweigten Fettsäuren mit Glycerin, aus verzweigten Alkanolen, z. B. Guerbet-Alkoholen mit einer einzigen Verzweigung am Kohlenstoffatom 2 wie 2-Hexyldecanol, 2-Octyldodecanol, Isotridecanol und Isohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethern oder Di-n-alkylethern mit jeweils insgesamt 12 - 24 C-Atomen, insbesondere Di-n-octylether (Cetiol^{®}OE Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol^{®} APM), PPG-14-Butylether (Ucon Fluid^{®} AP), PPG-15-Stearylether (Arlamol^{®} E), PPG-9-Butylether (Breox^{®} B25) und PPG-10-Butandiol (Macol^{®} 57). Die unpolare Ölkomponente kann ausgewählt sein aus flüssigen Paraffinölen, Isoparaffinölen, z. B. Isohexadecan und Isoeicosan, aus hydrogenierten Polyalkenen, insbesondere Poly-1-decenen (im Handel erhätlich als Nexbase 2004, 2006 oder 2008 FG (Fortum, Belgien)), aus synthetischen Kohlenwasserstoffen, z. B. 1,3-Di-(2-ethyl-hexyl)-cyclohexan (Cetiol^{®} S), sowie aus flüchtigen und nichtflüchtigen Siliconölen, die cyclisch, wie z. B. Decamethylcyclopentasiloxan und Dodecamethylcyclohexasiloxan, oder linear sein können, z. B. lineares Dimethylpolysiloxan, im Handel erhältlich z. B. unter der Bezeichnung Dow Corning^{®} 190, 200, 244, 245, 344 oder 345 und Baysilon^{®} 350 M. Geeignete Träger für die erfindungsgemäßen Zusammensetzungen sind bevorzugt wässrig.

Die erfindungsgemäßen Zusammensetzungen können aber auch im Wesentlichen wasserfrei sein, das heißt maximal 5 Gew.-%, bevorzugt maximal 1 Gew.-% Wasser enthalten.

In bevorzugten wässrigen Darreichungsformen beträgt der Wassergehalt bevorzugt 5 - 98 Gew.-%, mehr bevorzugt 10 - 90 und insbesondere 15 - 85 Gew.-%, bezogen auf die gesamte Zusammensetzung.

Die erfindungsgemäßen Zusammensetzungen können weiterhin wenigstens ein hydrophil modifiziertes Silicon enthalten. Sie ermöglichen die Formulierung hochtransparenter Zusammensetzungen, reduzieren die Klebrigkeit und hinterlassen ein frisches Hautgefühl. Unter hydrophil modifizierten Siliconen werden Polyorganosiloxane mit hydrophilen Substituenten verstanden, welche die Wasserlöslichkeit der Silicone bedingen. Unter Wasserlöslichkeit wird verstanden, dass sich wenigstens 2 Gew.-% des mit hydrophilen Gruppen modifizierten Silicons in Wasser bei 20 °C lösen. Entsprechende hydrophile Substituenten sind beispielsweise Hydroxy-, Polyethylenglycol- oder Polyethylenglycol/Polypropylenglycol-Seitenketten sowie ethoxylierte Ester-Seitenketten. Bevorzugt geeignet sind hydrophil modifizierte Silicon-Copolyole, insbesondere Dimethicone-Copolyole, die beispielsweise von Wacker-Chemie unter der Bezeichnung Belsil^{®} DMC 6031, Belsil^{®} DMC 6032, Belsil^{®} DMC 6038 oder Belsil^{®} DMC 3071 VP bzw. von Dow Corning unter der Bezeichnung DC 2501 im Handel sind. Besonders bevorzugt geeignet ist die Verwendung von Belsil^{®} DMC 6038, da es die Formulierung hochtransparenter Zusammensetzungen ermöglicht, die beim Verbraucher eine höhere Akzeptanz erreichen. Als hydrophiles Silikonderivat kann des weiteren etwa auch ABIL EM97 von Degussa/Goldschmidt eingesetzt werden. Es kann aber auch ein beliebiges Gemisch der genannten Silicone eingesetzt werden.

Die Menge des hydrophil modifizierten Silicons in den erfindungsgemäßen Zusammensetzungen beträgt bevorzugt 1 - 8 Gew.-% und besonders bevorzugt 2 - 6 Gew.-%, bezogen auf das Gesamtgewicht der Zusammensetzung.

In einer ebenfalls bevorzugten Ausführungsform handelt es sich bei der erfindungsgemäßen Zusammensetzung um ein Haarshampoo, eine Flüssigseife, ein Dusch- oder Waschgel oder ein Schaumbad. Innerhalb dieser Ausführungsform enthält die erfindungsgemäße Zusammensetzung bevorzugt mindestens ein anionisches, zwitterionisches, ampholytisches und/oder kationisches Tensid.

Bevorzugte anionische Tenside sind Alkylsulfate, Alkylpolyglycolethersulfate und Ethercarbonsäuren mit 10 bis 18 C-Atomen in der Alkylgruppe und bis zu 12 Glycolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglyceridsulfate, Alkyl- und Alkenyletherphosphate sowie Eiweißfettsäurekondensate.

Zwitterionische Emulgatoren tragen im Molekül mindestens eine quartäre Ammoniumgruppe und mindestens eine -COO⁻ - oder -SO₃⁻ -Gruppe. Besonders geeignete zwitterionische Emulgatoren sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Ampholytische Emulgatoren enthalten außer einer C₈ - C₂₄-Alkyl- oder -Acylgruppe mindestens eine freie Aminogruppe und mindestens eine -COOH- oder -SO₃H-Gruppe im Molekül und können innere Salze ausbilden. Beispiele für geeignete ampholytische Emulgatoren sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Die zuvor beschriebenen ionischen Tenside sind in den erfindungsgemäßen Zusammensetzungen dieser Ausführungsform bevorzugt in einer Menge von 0,05 bis 30 Gew.-% und besonders bevorzugt in einer Menge von 0,1 bis 20 Gew.-%, bezogen auf die gesamte Zusammensetzung, enthalten.

Erfindungsgemäß verwendbare nichtionische Tenside sind beispielsweise:
- alkoxylierte Fettsäurealkylester der Formel R¹CO-(OCH₂CHR²)ₓOR³, in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und x für Zahlen von 1 bis 20 steht,
- Anlagerungsprodukte von Ethylenoxid an Fettsäurealkanolamide und Fettamine,
- Fettsäure-N-alkylglucamide,
- C₈ - C₂₂-Alkylamin-N-oxide,
- Alkylpolygykoside entsprechend der allgemeinen Formel RO-(Z)ₓ wobei R für eine C₈- C₁₆-Alkylgruppe, Z für Zucker sowie x für die Anzahl der Zuckereinheiten steht. Die verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind solche Alkylpolyglykoside, bei denen R im wesentlichen aus C₈- und C₁₀-Alkylgruppen, im wesentlichen aus C₁₂- und C₁₄-Alkylgruppen, im wesentlichen aus C₈- bis C₁₆-Alkylgruppen oder im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglykoside enthalten im Schnitt 1,1 bis 5, bevorzugt 1,1 bis 2,0 besonders bevorzugt 1,1 bis 1,8 Zuckereinheiten. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

In einer vorteilhaften Ausführungsform enthalten die zuvor beschriebennen erfindungsgemäßen Zusammensetzungen mindestens ein filmbildendes, emulsionsstabilisierendes, verdickendes oder adhäsives Polymer, ausgewählt aus natürlichen und synthetischen Polymeren, die kationisch, anionisch, amphoter geladen oder nichtionisch sein können. Bevorzugt sind kationische, anionische sowie nichtionische Polymere.

Unter den kationischen Polymeren bevorzugt sind Polysiloxane mit quaternären Gruppen, z. B. die Handelsprodukte Q2-7224 (Dow Corning), Dow Corning^{®} 929 Emulsion (mit Amodimethicone), SM-2059 (General Electric), SLM-55067 (Wacker) sowie Abil^{®}-Quat 3270 und 3272 (Degussa). Bevorzugte anionische Polymere und nichtionische Polymere wurden an früherer Stelle bereits beschrieben.

Die zuvor beschriebenen erfindungsgemäßen Mittel können weitere Wirk-, Hilfs- und Zusatzstoffe enthalten, beispielsweise:
- Allantoin,
- Antioxidantien, zum Beispiel Imidazole (z. B. Urocaninsäure) und deren Derivate,
- Peptide wie D,L-Carnosin, D-Carnosin, L-Carnosin und deren Derivate (z. B. Anserin),
- Chlorogensäure und deren Derivate,
- Liponsäure und deren Derivate (z. B. Dihydroliponsäure),
- Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze,
- Dilaurylthiodipropionat, Distearylthiodipropionat, Thiodipropionsäure und deren Derivate (Ester, Ether, Peptide, Lipide, Nukleotide, Nukleoside und Salze) sowie
- Sulfoximinverbindungen (z. B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner
- (Metall)-Chelatoren (z. B. α-Hydroxyfettsäuren, Palmitinsäure, Phytinsäure, Lactoferrin),
- Huminsäure, Gallensäure, Gallenextrakte, Bilirubin, Biliverdin, EDTA, EGTA und deren Derivate,
- ungesättigte Fettsäuren und deren Derivate (z. B. γ-Linolensäure, Linolsäure, Ölsäure),
- Folsäure und deren Derivate,
- Ubichinon und Ubichinol und deren Derivate,
- das Koniferylbenzoat des Benzoeharzes,
- Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon,
- Harnsäure und deren Derivate, Katalase, Superoxid-Dismutase,
- Zink und dessen Derivate (z. B. ZnO, ZnSO₄),
- Selen und dessen Derivate (z. B. Selen-Methionin),
- Stilbene und deren Derivate (z. B. Stilbenoxid, trans-Stilbenoxid) und die als Antioxidans geeigneten Derivate (Salze, Ester, Ether, Zucker, Nukleotide, Nukleoside, Peptide und Lipide) dieser Wirkstoffe,
- Ceramide und Pseudoceramide,
- Triterpene, insbesondere Triterpensäuren wie Ursolsäure, Rosmarinsäure, Betulinsäure, Boswelliasäure und Bryonolsäure,
- Monomere Catechine, besonders Catechin und Epicatechin, Leukoanthocyanidine, Catechinpolymere (Catechin-Gerbstoffe) sowie Gallotannine,
- Verdickungsmittel, z. B. Gelatine, Pflanzengumme wie Agar-Agar, Guar-Gum, Alginate, Xanthan-Gum, Gummi arabicum, Karaya-Gummi oder Johannisbrotkernmehl, natürliche und synthetische Tone und Schichtsilikate, z. B. Bentonit, Hectorit, Montmorillonit oder Laponite^{®}, vollsynthetische Hydrokolloide wie z. B. Polyvinylalkohol, und außerdem Ca-, Mg- oder Zn-Seifen von Fettsäuren,
- Pflanzenglycoside,
- Strukturanten wie Maleinsäure und Milchsäure,
- Dimethylisosorbid,
- Alpha-, beta- sowie gamma-Cyclodextrine, insbesondere zur Stabilisierung von Retinol,
- Lösungsmittel, Quell- und Penetrationsstoffe wie Ethanol, Isopropanol, Ethylenglykol, Propylenglykol, Propylenglykolmonoethylether, Glycerin und Diethylenglykol, Carbonate, Hydrogencarbonate, Guanidine, Harnstoffe sowie primäre, sekundäre und tertiäre Phosphate
- Parfümöle, Pigmente sowie Farbstoffe zum Anfärben des Mittels,
- Substanzen zur Einstellung des pH-Wertes, z. B. α- und β-Hydroxycarbonsäuren,
- Komplexbildner wie EDTA, NTA, β-Alanindiessigsäure und Phosphonsäuren,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Treibmittel wie Propan-Butan-Gemische, N₂O, Dimethylether, CO₂ und Luft,
- MMP-1-inhibierende Substanzen, insbesondere ausgewählt aus Photolyase und/oder T4 Endonuclease V, Propylgallat, Precocenen, 6-Hydroxy-7-methoxy-2,2-dimethyl-1(2H)-benzopyran und 3,4-Dihydro-6-hydroxy-7-methoxy-2,2-dimethyl-1(2H)-benzopyran,
- organische, mineralische und/oder modifizierte mineralische Lichtschutzfilter, insbesondere UVA-Filter und/oder UVB-Filter.

In einer weiteren bevorzugten Ausführungsform können die erfindungsgemäßen Mittel als Mund-, Zahn- und/oder Zahnprothesenpflegemittel wie beispielsweise als Mundwasser, Gel, flüssige Zahnputzlotion, steife Zahnpaste, Kaugummi, Gebissreiniger oder Prothesenhaftcreme vorliegen. Insbesondere die Abtötung bzw. Hemmung der für die Mundflora schädlichen Keime (insbesondere Streptococcus mutans, S. salivarius, S. mitis, Porphyromonas gingivalis, Treponema denticola, Fusobacterium nucleatum, Actinomyces naeslundii) einhergehend mit einer überraschenderweise geringen bis nicht vorhandenen Wirkung auf die für die Mundflora positiven Keime (wie z.B. insbesondere *Streptococcus thermophilus)* führen dazu, dass erfindungsgemäße Zusammensetzungen für die Mund- und Zahnpflege hervorragend geeignet sind.

Hierzu ist es erforderlich, dass die erfindungemäßen Zusammensetzungen einen für diese Ausführungsform geeigneten Träger enthalten.

Als Träger können pulverförmige Zusammensetzungen oder wässrig-alkoholische Lösungen dienen, die als Mundwässer 0 bis 15 Gew.-% Ethanol, 1 bis 1,5 Gew.-% Aromaöle und 0,01 bis 0,5 Gew.-% Süßstoffe oder als Mundwasser-Konzentrate 15 bis 60 Gew.-% Ethanol, 0,05 bis 5 Gew.-% Aromaöle, 0,1 bis 3 Gew.-% Süßstoffe sowie ggf. weitere Hilfsstoffe enthalten können und vor Gebrauch mit Wasser verdünnt werden. Die Konzentration der Komponenten muss dabei so hoch gewählt werden, dass nach Verdünnung die genannten Konzentrationsuntergrenzen bei der Anwendung nicht unterschritten werden.

Als Träger können aber auch Gele sowie mehr oder weniger fließfähige Pasten dienen, die aus flexiblen Kunststoffbehältern oder Tuben ausgedrückt und mit Hilfe einer Zahnbürste auf die Zähne aufgetragen werden. Solche Produkte enthalten höhere Mengen an Feuchthaltemitteln und Bindemitteln oder Konsistenzreglern und Polierkomponenten. Darüber hinaus sind auch in diesen Zusammensetzungen Aromaöle, Süßstoffe und Wasser enthalten.

Als Feuchthaltemittel können dabei z.B. Glycerin, Sorbit, Xylit, Propylenglykole, Polyethenylenglycole oder Gemische dieser Polyole, insbesondere solche Polyethenylenglycole mit Molekulargewichten von 200 bis 800 (von 400 - 2000) verwendet werden. Bevorzugt ist als Feuchthaltemittel Sorbit in einer Menge von 25 - 40 Gew.-% enthalten.

Als Antizahnstein-Wirkstoffe und als Demineralisierungs-Inhibitoren können kondensierte Phosphate in Form ihrer Alkalisalze, bevorzugt in Form ihrer Natrium- oder Kaliumsalze enthalten sein. Die wäßrigen Lösungen dieser Phosphate reagieren aufgrund hydrolytischer Effekte alkalisch. Durch Säurezusatz wird der pH-Wert der Mund-, Zahn- und/oder Zahnprothesenpflegemittel auf die bevorzugten Werte von 7,5 - 9 eingestellt.

Es können auch Gemische verschiedener kondensierter Phosphate oder auch hydratisierte Salze der kondensierten Phosphate eingesetzt werden. Die spezifizierten Mengen von 2 - 12 Gew.-% beziehen sich jedoch auf die wasserfreien Salze. Bevorzugt ist als kondensiertes Phosphat ein Natrium- oder Kalium-tripolyphosphat in einer Menge von 5 - 10 Gew.-% der Zusammensetzung enthalten.

Ein bevorzugt enthaltener Wirkstoff ist eine karieshemmende Fluorverbindung, bevorzugt aus der Gruppe der Fluoride oder Monofluorophosphate in einer Menge von 0,1 - 0,5 Gew.-% Fluor. Geeignete Fluorverbindungen sind z.B. Natriummonofluorophosphat (Na₂PO₃F), Kaliummonofluorophosphat, Natrium- oder Kaliumfluorid, Zinnfluorid oder das Fluorid einer organischen Aminoverbindung.

Als Bindemittel und Konsistenzregler dienen z.B. natürliche und synthetische wasserlösliche Polymere wie Carragheen, Traganth, Guar, Stärke und deren nichtionogene Derivate wie z.B. Hydroxypropylguar, Hydroxyethylstärke, Celluloseether wie z.B. Hydroxyethylcellulose oder Methylhydroxypropylcellulose. Auch Agar-Agar, Xanthan-Gum, Pektine, wasserlösliche Carboxyvinylpolymere (z.B. Carbopol^{®}-Typen), Polyvinylalkohol, Polyvinylpyrrolidon, höhermolekulare Polyethylenglykole (Molekulargewicht 10³ bis 10⁶ D). Weitere Stoffe, die sich zur Viskositätskontrolle eignen, sind Schichtsilikare wie z.B. Montmorillonit-Tone, kolloidale Verdickungskieselsäuren, z.B. Aerogel-Kieselsäure oder pyrogene Kieselsäuren.

Als Polierkomponenten können alle hierfür bekannten Poliermittel, bevorzugt aber Fällungs- und Gelkieselsäuren, Aluminiumhydroxid, Aluminiumsilicat, Aluminiumoxid, Aluminiumoxidtrihydrat, unlösliches Natriummetaphosphat, Calciumpyrophosphat, Calciumhydrogenphosphat, Dicalciumphosphat, Kreide, Hydroxylapatit, Hydrotalcite, Talkum, Magnesiumaluminiumsilicat (Veegum^{®}), Calciumsulfat, Magnesiumcarbonat, Magnesiumoxid, Natriumaluminiumsilikate, z.B. Zeolith A oder organische Polymere, z.B. Polymethacrylat, eingesetzt werden. Die Poliermittel werden vorzugsweise in kleineren Mengen von z.B. 1 - 10 Gew.-% verwendet.

Die erfindungsgemäßen Zahn- und/oder Mundpflegeprodukte können durch Zugabe von Aromaölen und Süßungsmitteln in ihren organoleptischen Eigenschaften verbessert werden. Als Aromaöle kommen alle für Mund-, Zahn- und/oder Zahnprothesenpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Kümmelöl, Eukalyptusöl, Fenchelöl, Zimtöl, Geraniumöl, Salbeiöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton.

Als Süßungsmittel eignen sich entweder natürliche Zucker wie Sucrose, Maltose, Lactose und Fructose oder synthetische Süßstoffe wie z.B. Saccharin-Natriumsalz, Natriumcyclamat oder Aspartam.

Als Tenside sind dabei insbesondere Alkyl- und/oder Alkenyl-(oligo)-glycoside einsetzbar. Bezüglich des Glycosidrestes gilt, daß sowohl Monoglycoside (x = 1), bei denen ein Pentose- oder Hexoserest glycosidisch an einen primären Alkohol mit 4 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl- und/oder Alkenyl-(oligo)-glycosid ein Alkyl- und/oder Alkenyl-(oligo)-glucosid der Formel RO(C₆H₁₀O)ₓ-H, in der R eine Alkyl- und/oder Alkenyl-gruppe mit 8 bis 14 C-Atomen ist und x einen Mittelwert von 1 bis 4 hat. Besonders bevorzugt sind Alkyloligoglucoside auf Basis von gehärtetem C_{12/14}-Kokosalkohol mit einem DP von 1 bis 3. Das Alkyl- und/oder Alkenyl-glycosid-Tensid kann sehr sparsam verwendet werden, wobei bereits Mengen von 0,005 bis 1 Gew.-% ausreichend sind.

Außer den genannten Alkylglucosid-Tensiden können auch andere nichtionische, ampholytische und kationische Tenside enthalten sein, als da beispielsweise sind: Fettalkoholpolyglycolethersulfate, Monoglyceridsulfate, Monoglyceridethersulfate, Mono- und/oder Dialkylsulfosuccinate, Fettsäureisethionate, Fettsäuresarcosinate, Fettsäuretauride, Fettsäureglutamate, Ethercarbonsäuren, Fettsäureglucamide, Alkylamido-betaine und/oder Proteinfettsäurekondensate, letztere vorzugsweise auf Basis von Weizenproteinen. Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäuresorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

Die erfindungsgemäßen Mund-, Zahn- und/oder Zahnprothesenpflegemittel enthalten bevorzugt als Lösungsvermittler für gegebenenfalls enthaltene Aromaöle Anlagerungsprodukte von 20 bis 60 Mol Ethylenoxid an gehärtetes oder ungehärtetes Rizinusöl (d.h. an Oxystearinsäure- oder Ricinolsäure-triglycerid), an Glyzerin-mono- und/oder -distearat oder an Sorbitanmono- und/oder - distearat.

Weitere übliche Zusätze für die Mund-, Zahn- und/oder Zahnprothesenpflege mittel sind z.B.
- Pigmente, z.B. Titandioxid, und/oder Farbstoffe
- pH-Stellmittel und Puffersubstanzen wie z.B. Natriumbicarbonat, Natriumcitrat, Natriumbenzoat, Zitronensäure, Phosphorsäure oder saure Salze, z.B. NaH₂PO₄
- wundheilende und entzündungshemmende Stoffe wie z.B. Allantoin, Harnstoff, Panthenol, Azulen bzw. Kamillenextrakt
- weitere gegen Zahnstein wirksame Stoffe wie z.B. Organophosphonate, z.B. Hydroxyethandiphosphonate oder Azacycloheptandiphosphonat
- Konservierungsstoffe wie z.B. Sorbinsäure-Salze, p-Hydroxybenzoesäure-Ester.
- Plaque-Inhibitoren wie z.B. Hexachlorophen, Chlorhexidin, Hexetidin, Triclosan, Bromchlorophen, Phenylsalicylsäureester.
In einer besonderen Ausführungsform kann die erfindungsgemäße Zusammensetzung ferner eine Mundspülung, ein Mundwasser, ein Prothesenreiniger oder ein Prothesenhaftmittel sein.

Für erfindungsgemäß bevorzugten Prothesenreiniger, insbesondere Prothesenreinigungstabletten und -pulver, eignen sich neben den schon genannten Inhaltsstoffen für die Mund-, Zahn- und/oder Zahnprothesenpflege zusätzlich noch Per-Verbindungen wie beispielsweise Peroxoborat, Peroxomonosulfat oder Percarbonat. Sie haben den Vorteil, dass sie neben der Bleichwirkung gleichzeitig auch desodorierend und/oder desinfizierend wirken. Der Einsatz solcher Per-Verbindungen in Prothesenreinigern beträgt zwischen 0,01 und 10 Gew.-%, insbesondere zwischen 0,5 und 5 Gew.-%.

Als weitere Inhaltsstoffe sind auch Enzyme, wie z.B. Proteasen und Carbohydrase, zum Abbau von Proteinen und Kohlenhydraten geeignet. Der pH-Wert kann zwischen pH 4 und pH 12, insbesondere zwischen pH 5 und pH 11 liegen.

Für die Prothesenreinigungstabletten sind zusätzlich noch weitere Hilfsstoffe notwendig, wie beispielsweise Mittel, die einen sprudelnden Effekt hervorrufen, wie z.B. CO₂ freisetzende Stoffe wie Natriumhydrogencarbonat, Füllstoffe, z.B. Natriumsulfat oder Dextrose, Gleitmittel, z.B. Magnesiumstearat, Fließregulierungsmittel, wie beispielsweise kolloidales Siliziumdioxid und Granuliermittel, wie die bereits erwähnten hochmolekularen Polyethylenglykole oder Polyvinylpyrrolidon.

Prothesenhaftmittel können als Pulver, Cremes, Folien oder Flüssigkeiten angeboten werden und unterstützen die Haftung der Prothesen.

Als Wirkstoffe sind natürliche und synthetische Quellstoffe geeignet. Als natürliche Quellstoffe sind neben Alginaten auch Pflanzengummen, wie z.B. Gummi arabicum, Traganth und Karaya-Gummi sowie natürlicher Kautschuk aufzufassen. Insbesondere haben sich Alginate und synthetische Quellstoffe, wie z.B. Natriumcarboxymethylcellulose, hochmolekulare Ethylenoxid-Copolymere, Salze der Poly(vinyl-ether-co-maleinsäure) und Polyacrylamide.

Als Hilfsstoffe für pastöse und flüssige Produkte eignen sich besonders hydrophobe Grundlagen, insbesondere Kohlenwasserstoffe, wie beispielsweise Weißes Vaselin (DAB) oder Paraffinöl.

Ein zweiter Gegenstand der Erfindung ist die kosmetische Verwendung einer Wirkstoffkombination aus
a) 0,000005 bis 0,005 Gew.-% mindestens einer funktionalisierten Verbindung aus zwei bis sechs über Amidbindungen miteinander verknüpften alpha-Aminosäuren und
b) 0,0001 bis 10 Gew.-% mindestens eines Extrakts aus Pflanzen der Familie der Monimiaceae.

in kosmetischen Haut- und/oder Haarbehandlungsmitteln zur Bekämpfung von Mikroorganismen. Unter den vorgenannten Mikroorganismen sind Bakterien, Hefen, andere Pilzarten und weitere unerwünschte Keine zu verstehen.

Bei den unerwünschten Keimen handelt es sich vorzugsweise um hautfeindliche Keime und/oder pathogene Keime, besonders bevorzugt um koagulase-positive Staphylokokken, insbesondere S. aureus oder um gram-negative Bakterien, bevorzugt Pseudomonaden, insbesondere P. aeruginosa, und besonders bevorzugt, um geruchsbildende Keime, insbesondere geruchsbildende Staphylokokken, vor allem um *S. hominis,* um geruchsbildende gram-positive anaerobe Kokken, insbesondere Peptostreptokokken, vor allem um *Anaerococcus octavius*, und/oder um geruchsbildende Corynebakterien und/oder um geruchsbildende Mikrokokken, vor allem *Micrococcus luteus*.

Bei den im Oralbereich unerwünschten Keimen handelt es sich vorzugsweise um kariogene Bakterien, besonders bevorzugt um Streptokken, insbesondere *Streptococcus mutans, S. salivarius* und *S. mitis,* um gram-negative Erreger von Gingivitiden, insbesondere um *Porphyromonas gingivalis, Treponema denticola, Fusobacterium nucleatum* und *Actinomyces naeslundii*.

Insbesondere werden unter hautfeindlichen und/oder pathogenen Keimen Koagulase-positive Staphylokokken, insbesondere Staphylococcus aureus, oder Keime ausgewählt aus der Gruppe bestehend aus Propionbakterium acnes, Candida albicans, Malassezia furfur, Corynebacterium spp. oder Peptostreptococcus spp., insbesondere Propionbacterium acnes, verstanden.

Im Falle von Mund- und/oder Zahnpflegeprodukten werden unter unerwünschten Keimen weiterhin Streptococcus-Bakterien, insbesondere Streptococcus mutans, Streptococcus grodonii, A. naeslundii und/oder Fusobacterium nucleatum verstanden.

Im Fall von (unangenehmem) Körpergeruch, und insbesondere Achselgeruch, handelt es sich bei den unerwünschten Keimen nicht notwendigerweise um pathogene Keime, sondern bei den geruchsbildenden Keimen kann es sich natürlich ebenso um an und für sich hautfreundliche Keime handeln. Im Falle von (unangenehmem) Körpergeruch sind die unerwünschten Keime also dadurch definiert, dass sie Körpergeruch verursachen.

Unter "geruchsbildenden Keimen" bzw. "Geruchskeimen" sind grundsätzlich solche Mikroorganismen zu verstehen, die bei Menschen mit Körpergeruch vermehrt auftreten. Bevorzugt handelt es sich hierbei um Mikroorganismen, die entweder selbst Substanzen produzieren bzw. die Bildung von Substanzen fördern, die einen unangenehmen Geruch verursachen. Des weiteren kann es sich hierbei auch um Mikroorganismen handeln, die nur mittelbar in die Bildung solcher Substanzen involviert sind, beispielsweise dadurch, dass sie eine Substanz produzieren bzw. die Bildung von Substanzen fördern, die durch andere Mikroorganismen zu unangenehm riechenden Substanzen umgesetzt werden können. Die geruchsbildenden Mikroorganismen müssen also erfindungsgemäß nicht notwendigerweise selbst den unangenehmen Geruch verursachen, sondern können auch in anderer Weise in den Metabolismus der Geruchsbildung involviert sein. Ein dritter Gegenstand der Erfindung ist die kosmetische Verwendung einer Wirkstoffkombination aus
a) 0,000005 bis 0,005 Gew.-% mindestens einer funktionalisierten Verbindung aus zwei bis sechs über Amidbindungen miteinander verknüpften alpha-Aminosäuren und
b) 0,0001 bis 10 Gew.-% mindestens eines Extrakts aus Pflanzen der Familie der Monimiaceae.
in kosmetischen Haut- und/oder Haarbehandlungsmitteln zur Stimulierung der humanen Haupeptide β-Defensine-2 und β-Defensine-3.

Ein vierter Gegenstand der Erfindung ist ein Verfahren zur kosmetischen Behandlung der Haut und/oder der Haare, bei dem eine erfindungsgemäße Zusammensetzung auf die Haut und/oder die Haare aufgebracht und nach einer Einwirkungszeit von 5 Sekunden bis 5 Minuten wieder ausgespült wird.

Ein fünfter Gegenstand der Erfindung ist ein Verfahren zur kosmetischen Behandlung der Haut und/oder der Haare, bei dem eine erfindungsgemäße Zusammensetzung auf die Haut und/oder die Haare aufgebracht und bis zur nächsten Reinigung auf der Haut oder den Haaren belassen wird.

Vorzugsweise ist die erfindungsgemäße Zusammensetzung dazu geeignet, das natürlicherweise vorkommende gesunde mikrobielle Gleichgewicht der Hautflora wiederherzustellen bzw. zu stabilisieren.

Die Behandlung kann hierbei jeweils auch präventiv bzw. prophylaktisch erfolgen.

Beispiele für Öl-in-Wasser-Emulsionen:

**1. Beispielserie:**

| | **1** | **2** | **3** |
|---|---|---|---|
| Distelöl | 3,00 | 3,00 | 3,00 |
| Myritol^{®} 318 | 5,00 | 5,00 | 5,00 |
| Novata^{®} AB | 2,00 | 2,00 | 2,00 |
| Lanette^{®} 22 | 1,00 | 1,00 | 1,00 |
| Cutina^{®} MD | 2,00 | 2,00 | 2,00 |
| Stenol^{®} 1618 | 1,00 | 1,00 | 1,00 |
| Baysilon^{®} M350 | 1,00 | 1,00 | 1,00 |
| Controx^{®} KS | 0,05 | 0,05 | 0,05 |
| Propylparaben | 0,20 | 0,20 | 0,20 |
| Dry Flo^{®} Plus | 1,00 | 2,00 | 3,00 |
| Propylenglycol | 5,00 | 5,00 | 5,00 |
| Glycerine | 5,00 | 3,00 | 3,00 |
| Methylparaben | 0,20 | 0,20 | 0,20 |
| Phenoxyethanol | 0,90 | 0,50 | 0,90 |
| Parfum | 0,10 | 0,10 | 0,10 |
| Bodyfensine^{®} | 2 | 1 | 0,5 |
| Betapur^{®} | 1 | 2 | 0,5 |
| Aqua | ad.100 | ad.100 | ad.100 |

**2. Beispielserie:**

| | **1** | **2** | **3** |
|---|---|---|---|
| Lipoid^{®} S75-3 | 0,50 | 0,50 | 0,50 |
| Tocoperylacetat | 0,50 | 0,50 | 0,50 |
| Cutina^{®} MD | 1,00 | 1,00 | 1,00 |
| Lanette^{®} 22 | 2,00 | 2,00 | 2,00 |
| Baysilon^{®} M 350 | 0,50 | 0,50 | 0,50 |
| Propylparaben | 0,20 | 0,20 | 0,20 |
| Dow Corning^{®} 9040 | 1,00 | 1,00 | 1,00 |
| Glycerin | 4,50 | 3,00 | 3,00 |
| Hexandiol | 6,00 | 3,00 | |
| Methylparaben | 0,20 | 0,20 | 0,20 |
| Tego Carbomer^{®} 140 | 0,30 | 0,30 | 0,30 |
| DS-HCN^{®} | 5,00 | 5,00 | 5,00 |
| Propylenglycol | 5,00 | 5,00 | 5,00 |
| Simulgel^{®} NS | 1,50 | 1,50 | 1,50 |
| TiO₂ | 0,50 | 0,50 | 0,50 |
| Bodyfensine^{®} | 2 | 1 | 0,5 |
| Betapur^{®} | 1 | 2 | 0,5 |
| Aqua | ad.100 | ad.100 | ad.100 |

**3. Beispielserie:**

| | **1** | **2** | **3** |
|---|---|---|---|
| Sisterna^{®} SP30-C | 2,00 | 2,00 | 2,00 |
| Sisterna^{®} SP70-C | 0,80 | 0,80 | 0,80 |
| Propylparaben | 0,20 | 0,20 | 0,20 |
| Methylparaben | 0,20 | 0,20 | 0,20 |
| Ethanol | 5,00 | 3,00 | |
| Dry Flo^{®} Plus | 1,00 | 1,00 | 1,00 |
| Parfum | 0,30 | 0,30 | 0,30 |
| Cetearyl Alkohol | 1,00 | 1,00 | 1,00 |
| Fucogel^{®} 1000 | 2,00 | 1,00 | 0,50 |
| Keltrol^{®} SF | 0,20 | | 0,20 |
| Aristoflex^{®} AVC | | 0,50 | |
| Hexandiol-1,6 | 5,00 | | |
| Glycerine | 10,00 | 5,00 | 10,00 |
| Dow Corning^{®} 200 Fluid | 7,00 | | |
| Dow Corning^{®} 245 | | 5,00 | 5,00 |
| Cetiol^{®} 868 | 2,00 | | 2,00 |
| Bodyfensine^{®} | 2 | 1 | 0,5 |
| Betapur^{®} | 1 | 2 | 0,5 |
| Aqua | ad.100 | ad.100 | ad.100 |

**4. Beispielserie:**

| | **1** | **2** | **3** |
|---|---|---|---|
| Dow Corning^{®} 245 | 25,00 | 25,00 | 25,00 |
| Abil^{®} EM 90 | 2,00 | 2,00 | 2,00 |
| Belsil^{®} DM 100 | 2,50 | 2,50 | 2,50 |
| Hydrogenated Castor Oil | 2,00 | 2,50 | 2,00 |
| Glycerine | 5,00 | 5,00 | 5,00 |
| NaCl | 2,00 | 2,00 | 2,00 |
| Symdiol^{®} 68 | 0,50 | 0,50 | 0,50 |
| Phenoxyethanol | 0,40 | 0,40 | 0,40 |
| Parfum | 0,30 | 0,30 | 0,30 |
| Bodyfensine^{®} | 2 | 1 | 0,5 |
| Betapur^{®} | 1 | 2 | 0,5 |
| Aqua | ad.100 | ad.100 | ad.100 |

**5. Beispielserie**

| | **1** | **2** | **3** |
|---|---|---|---|
| Montanov^{®} 202 | 5,00 | 5,00 | 5,00 |
| Cutina^{®} MD | 2,00 | 2,00 | 2,00 |
| Cetearyl Alkohol | 1,00 | 1,00 | 1,00 |
| Cetiol^{®} B | 9,00 | 9,00 | 9,00 |
| Kontrox^{®} KS | 0,05 | 0,05 | 0,05 |
| Dow Corning^{®} 245 | | 7,00 | 3,00 |
| Dow Corning^{®} 9040 | 1,00 | 1,00 | |
| Parfum | 0,30 | 0,30 | 0,30 |
| Fucogel^{®} 1000 | | 2,00 | |
| Keltrol^{®} SF | | 0,20 | 0,20 |
| Aristoflex^{®} AVC | | | 0,50 |
| Hexandiol-1,6 | 6,00 | 6,00 | |
| Glycerine | 3,00 | 5,00 | 10,00 |
| Dow Corning^{®} 200 Fluid | 7,00 | 7,00 | |
| Dow Corning^{®} 245 | | 5,00 | 3,00 |
| Dow Corning^{®} 9040 | 1,00 | | 1,00 |
| Propylenglycol | 2,00 | 2,00 | |
| Tego Carbomer^{®} | 0,30 | 0,30 | 0,30 |
| Dry Flo^{®} Plus | 1,00 | 1,00 | 1,00 |
| Bodyfensine^{®} | 2 | 1 | 0,5 |
| Betapur^{®} | 1 | 2 | 0,5 |
| Aqua | ad.100 | ad.100 | ad.100 |

**6. Beipsielserie**

| | **1** | **2** | **3** |
|---|---|---|---|
| Glucamate^{®} SSE-20 | 2,00 | 2,00 | 2,00 |
| Sympatens^{®} BS/080 | 2,00 | 2,00 | 2,00 |
| Dry Flo^{®} Plus | | 1,00 | 1,00 |
| Parfum | 0,30 | 0,30 | 0,30 |
| Silikonöl^{®} 350 | 0,30 | 0,30 | |
| Cutina^{®} MD | 2,00 | 2,00 | 2,00 |
| Paraffinöl | 2,00 | 1,00 | |
| Cetearyl Alkohol | 1,00 | 1,50 | 1,25 |
| Capryl-/Caprinsäure-triglycerid | | 3,50 | 3,00 |
| Phenoxyethanol | 0,90 | 0,50 | 0,90 |
| Keltrol^{®} SF | | | 0,20 |
| Aristoflex^{®} AVC | | 0,50 | 0,50 |
| Hexandiol-1,6 | | 5,00 | 5,00 |
| Glycerine | 2,00 | 5,00 | 10,00 |
| Culminal^{®} MHPC 100 | 0,10 | 0,10 | 0,10 |
| Propylenglycol | 2,00 | 5,00 | 3,00 |
| Bodyfensine^{®} | 2 | 1 | 0,5 |
| Betapur^{®} | 1 | 2 | 0,5 |
| Aqua | ad.100 | ad.100 | ad.100 |

**7 Beispielserie:**

| | **1** | **2** | **3** | **4** |
|---|---|---|---|---|
| Emulsiphos^{®} 677660 | 3,00 | 3,00 | 3,00 | 3,00 |
| Cetiol^{®} B | 5,00 | 5,00 | 5,00 | 5,00 |
| Estasan^{®} GT8-60 3575 MCT | 3,50 | 3,50 | 3,50 | 3,50 |
| Oil | | | | |
| Safloröl raffiniert | 2,00 | 2,00 | 2,00 | 2,00 |
| Behenylalkohol | 3,00 | 3,00 | 3,00 | 3,00 |
| Cetiol Sensoft | 1,00 | 1,00 | 1,00 | 1,00 |
| Vitamin A-acetat | 0,50 | 0,50 | 0,50 | 0,50 |
| Kontrox^{®} KS | 0,05 | 0,05 | 0,05 | 0,05 |
| Bienenwachs | 0,20 | 0,20 | 0,20 | 0,20 |
| Cosmedia^{®} SP | 0,50 | 0,50 | 0,50 | 0,50 |
| Butylmethoxydibenzoylmethan | 3,00 | 3,00 | 3,00 | 3,00 |
| Parsol^{®} SLX | 3,00 | 3,00 | 3,00 | 3,00 |
| Propylparaben | 0,20 | 0,20 | 0,20 | 0,20 |
| Hexandiol-1,6 | 5,00 | 5,00 | 5,00 | 5,00 |
| Glycerin 86%, pflanzlich | 4,50 | 4,50 | 4,50 | 4,50 |
| Sorbit 70% LS DAB | 2,00 | 2,00 | 2,00 | 2,00 |
| Methylparaben | 0,20 | 0,20 | 0,20 | 0,20 |
| Tego Carbomer^{®} 140 | 0,50 | 0,50 | 0,50 | 0,50 |
| NTA-Na3^{®} flüssig 40% | 0,10 | 0,10 | 0,10 | 0,10 |
| Cosmedia^{®} Silk | 2,00 | 2,00 | 2,00 | 2,00 |
| AMP^{®} Ultra PC 1000 | 0,90 | 0,90 | 0,90 | 0,90 |
| Neo Heliopan^{®} Hydro | 3,00 | 3,00 | 3,00 | 3,00 |
| Natriumhydroxid Perlen | 0,365 | 0,365 | 0,365 | 0,365 |
| Bodyfensine^{®} | 2,00 | 1,00 | 0,50 | 1,50 |
| Betapur^{®} | 1,00 | 2,00 | 0,50 | 1,50 |
| Aqua | ad.100 | ad.100 | ad.100 | ad.100 |

**Handelsprodukte:**

| Handelsname | INCI-Bezeichnung | Lieferant/Hersteller |
|---|---|---|
| Abil EM 90 | Cetyl PEG/PPG-10/1 Dimethicone | Degussa |
| AMP Ultra PC 1000 | Aminoethylpropanol | Dow |
| Aristoflex^{®} AVC | Ammonium Acryloyldimethyltaurate/VP Copolymer, t-Butyl alcohol | Clariant |
| Baysilon^{®} M350 | Dimethicone | Momentive |
| Belsil DM 100 | Polydimethylsiloxan | Wacker |
| Betapur^{®} | Water, Butylene Glycol, Peumus Boldus | BASF |
| | Leaf Extract, Xanthan Gum | |
| Bodyfensine^{®} | Water, Butylene Glycol, Acetyl Dipeptide-3 Aminohexanoate | Lipotec |
| Cetiol^{®} B | Dibutyladipate | Cognis |
| Cetiol^{®} Sensoft | Propylheptyl Caprylate | Cognis |
| Cetiol^{®} 868 | Ethylhexyl Stearate | Cognis |
| Controx KS | Tocopherol, Hydrogenated Palm Glycerides Citrate | Cognis |
| Cosmedia^{®} SP | Sodium Polyacrylate | Cognis |
| Culminal^{®} MHPC 100 | Methylhydroxypropylcellulose | Aqualon |
| Cutina MD | Glyceryl Stearate | Cognis |
| Dow Corning 200 | Dimethylpolysiloxan | Dow Corning |
| Dow Corning 9040 | Cyclomethicone, Dimethicone Crosspolymer | Dow Corning |
| Dow Corning 245 Fluid | Cyclomethicone, | Dow Corning |
| Dry Flo Plus | Aluminium Starch Octenylsuccinate | National Starch |
| DSH CN | Water, Dimethylsilanol Hyaluronate | Exsymol |
| Emulsiphos 677660 | Potassium Cetyl Phosphate, Hydrogenated Palm Glycerides | Symrise |
| Estasan GT-60 3575 | Caprylic/Capric Triglyceride | Croda |
| MCT Oil | | |
| Fucogel 1000 | Biosaccharide Gum-1 | Erbslöh |
| Glucamate SSE-20 | PEG-20 Methyl Glucose Sesquistearate | Noveon |
| Keltrol SF | Xanthan Gum | CP Kelco |
| Lanette^{®} 22 | Behenyl Alcohol | Cognis |
| Lipoid S75-3 | Hydrogenated Lecithin | Lipoid GmbH |
| Montanov 68 | Arachidyl Alcohol, Behenyl Ancohol, Arachidyl Glucoside | Seppic |
| Myritol 318 | Caprylic/Capric Triglyceride | Cognis |
| Novata AB | Cocoglycerides | Cognis |
| Neo Heliopan Hydro | Phenylbenzimidazole Sulfonic Acid | Symrise |
| NTA-Na₃ flüssig 40% | Nitrilotriacetic acid trisodium salt | Rexolin |
| Parsol SLX | Polysilicone-15 | L.C. United |
| Pemulen TR 1 | Acrylates/C10-30 Alkyl Acrylate Cross- polymer | |
| Simulgel NS | Hydroxyethyl Acrylate / Sodium Acryloyl- dimethyl Taurate Copolymer / Squalane / Polysorbate 60 | Seppic |
| Sisterna SP30-C | Sucrose Distearate | Sisterna |
| Sisterna SP70-C | Sucrose Stearate | Sisterna |
| Stenol 1618 | Cetearyl alcohol | Cognis |
| Symdiol 68 | 1,2-Hexanediol, Caprylyl Glycol | Symrise |
| Sympatens-BS/080 | PEG-8 Stearate | Dr. W. Kolb |
| Tego Carbomer 140 | Carbomer | Degussa |

## Patentansprüche

1. Zusammensetzung zur Behandlung der Haut und/oder der Haare, enthaltend in einem kosmetisch akzeptablen Träger - bezogen auf ihr Gesamtgewicht -
a) 0,000005 bis 0,005 Gew.-% mindestens einer funktionalisierten Verbindung aus zwei bis sechs über Amidbindungen miteinander verknüpften alpha-Aminosäuren und
b) 0,0001 bis 10 Gew.-% mindestens eines Extrakts aus Pflanzen der Familie der Monimiaceae.

2. Zusammensetzung nach Anspruch 1, **dadurch gekennzeichnet, dass** die funktionalisierte Verbindung a) am N-Terminus eine Acylierung und/oder am C-Terminus eine Amidierung aufweist.

3. Zusammensetzung gemäß einem der Ansprüche 1 oder 2, **dadurch gekennzeichnet, dass** sie zusätzlich mindestens einen Sebumregulator enthält.

4. Zusammensetzung gemäß einem der Ansprüche 1 bis 3, **dadurch gekennzeichnet, dass** sie zusätzlich mindestens einen antiinflammatorischen Wirkstoff enthält.

5. Zusammensetzung gemäß einem der Ansprüche 1 bis 4, **dadurch gekennzeichnet, dass** sie ein Wasch- und Reinigungsmittel für die Haut und/oder die Haare, ein Deodorant, ein Mund- und/oder Zahnpflegemittel, ein Hautpflegemittel und/oder ein Haarstylingmittel ist.

6. Verwendung einer Wirkstoffkombination, enthaltend
a) 0,000005 bis 0,005 Gew.-% mindestens einer funktionalisierten Verbindung aus zwei bis sechs über Amidbindungen miteinander verknüpften alpha-Aminosäuren und
b) 0,0001 bis 10 Gew.-% mindestens eines Extrakts aus Pflanzen der Familie der Monimiaceae.
in kosmetischen Haut- und/oder Haarbehandlungsmitteln zur Bekämpfung von Mikroorganismen.

7. Verwendung einer Wirkstoffkombination, enthaltend
a) 0,000005 bis 0,005 Gew.-% mindestens einer funktionalisierten Verbindung aus zwei bis sechs über Amidbindungen miteinander verknüpften alpha-Aminosäuren und
b) 0,0001 bis 10 Gew.-% mindestens eines Extrakts aus Pflanzen der Familie der Monimiaceae.
in kosmetischen Haut- und/oder Haarbehandlungsmitteln zur Stimulierung der humanen Hautpeptide β-Defensine-2 und β-Defensine-3.

8. Verfahren zur kosmetischen Behandlung der Haut und/oder der Haare, **dadurch gekennzeichnet, dass** eine Zusammensetzung nach einem der Ansprüche 1 bis 5 auf die Haut und/oder die Haare aufgebracht und nach einer Einwirkungszeit von 5 Sekunden bis 5 Minuten wieder ausgespült wird.

9. Verfahren zur kosmetischen Behandlung der Haut und/oder der Haare, **dadurch gekennzeichnet, dass** eine Zusammensetzung nach einem der Ansprüche 1 bis 5 auf die Haut und/oder die Haare aufgebracht und bis zur nächsten Reinigung auf der Haut oder den Haaren belassen wird.
